# EUROPEAN PATENT SPECIFICATION

(11) **EP 2 699 223 B1**
(45) Date of publication and mention of the grant of the patent: **28.02.2018**
(21) Application number: 12720622.5
(22) Date of filing: 16.04.2012
(51) Int. Cl.: A61K 8/64, A61Q 3/00, A61Q 7/00, A61K 8/67, A61Q 5/00

(54) **NEW COSMETIC USES OF GHK TRIPEPTIDE**
NEUE KOSMETISCHE VERWENDUNGEN DES GHK-TRIPEPTIDES
NOUVELLES UTILISATIONS COSMÉTIQUES DE TRIPEPTIDE GHK

(30) Priority: 21.04.2011 FR 1153470
(43) Date of publication of application: 26.02.2014
(73) Proprietor: Sederma, 78610 Le Perray en Yvelines (FR)
(72) Inventor: FOURNIAL, Arnaud, F-75016 Paris (FR); BERNARD, Gérard, F-92500 Rueil-Malmaison (FR)
(86) International application number: PCT/IB2012/051889
(87) International publication number: WO 2012/143845

(56) References cited:
- FR-A1- 2 791 684
- US-A1- 2003 007 938
- US-A1- 2004 132 667
- US-A1- 2006 067 905
- US-A1- 2010 249 042

## Description

### TECHNICAL FIELD

The present invention relates to new cosmetic uses of the Gly-His-Lys (GHK) tripeptide, derivatives or analogs.

The present invention relates more particularly to the fields of cosmetics, and hygiene and personal care products for mammals, animals or humans.

### BACKGROUND ART

The sequence of the Glycyl-histidyl-lysine peptide belongs to the family of matrikines. The various forms of GHK, including Palmitoyl-GHK or biotinoyl-GHK, have in common to actively participate in the reconstruction of the extracellular matrix of connective tissue, stimulate the synthesis of macromolecules, even to promote healing.

For example, the combination of biotinoyl-GHK, apigenin and oleanolic acid is used to fight the process of scalp hair aging for preventing scalp hair loss (Procapil™, product sold by Sederma).

The Pal-GHK is also used in combination with the Pal-GQPR (SEQ ID NO: 1) as a cosmetic anti-aging active ingredient (Matrixyl 3000™, product sold by Sederma). The association Pal-GHK, Pal-GQPR (SEQ ID NO: 1), N-hydroxysuccinimide and chrysin is also used as an active ingredient against under eye dark circles (Haloxyl™, product sold by Sederma).

### SUMMARY OF THE INVENTION

The present invention relates to the use of an effective amount of the tripeptide of formula R₁-Gly-His-Lys-R₂ wherein:
- R₁ is an hydrogen atom or an acyl group or a sulfonyl group selected from a biotinoyle or a group comprising an alkyl, aryl, aralkyl, sugar or alkoxy chain of 1 to 24 carbon atoms, which can be linear, branched or cyclic, saturated or unsaturated, hydroxylated or non-hydroxylated, sulfurated or non sulfurated, and
- R₂ is a hydroxyl function or a -O-R₃ group or a -NR₄R₅ group, R₃, R₄ and R₅ being independently of each other hydrogen, an alkyl, aryl, aralkyl, acyl, sulfonyl, sugar or alkoxy chain of 1 to 24 carbons, linear, branched or cyclic, substituted or unsubstituted, saturated or unsaturated, hydroxylated or non-hydroxylated, sulfurated or non sulfurated,
for the topical cosmetic non-therapeutical treatment
of eyelashes. The application also describes a topical composition comprising, in a physiologically acceptable medium, an effective amount of the tripeptide of formula R₁-Gly-His-Lys-R₂ wherein:
- R₁ is an hydrogen atom or an acyl group or a sulfonyl group selected from a biotinoyle or a group comprising an alkyl, aryl, aralkyl, sugar or alkoxy chain of 1 to 24 carbon atoms, which can be linear, branched or cyclic, saturated or unsaturated, hydroxylated or non-hydroxylated, sulfurated or non sulfurated, and
- R₂ is a hydroxyl function or a -O-R₃ group or a -NR₄R₅ group, R₃, R₄ and R₅ being independently of each other hydrogen, an alkyl, aryl, aralkyl, acyl, sulfonyl, sugar or alkoxy chain of 1 to 24 carbons, linear, branched or cyclic, substituted or unsubstituted, saturated or unsaturated, hydroxylated or non-hydroxylated, sulfurated or non sulfurated,
for the therapeutical treatment of nails and hair, excluding scalp hair.

Preferably, according to the invention, the Biot-GHK (R₁ is a biotinoyl group (also called biotinyl) and R₂ is a hydroxyl) or the Pal-GHK (R₁ is a palmitoyl group, that is to say an alkyl chain with 16 carbon atoms and R₂ is a hydroxyl) are used, which are commercial products. The Biot-GHK has the added benefit of providing together with the peptide part a vitamin-enriched part, biotin, for a healthy growth of hair and nails.

According to the invention, hair designates eyelashes. The treatment of the invention will include improving the appearance (length and thickness) of hair and their anchorage.

The GHK peptide will act at the hair follicle level and thus improve the anchoring of the hair and prevent loss of hair. It also helps get longer hair, thicker (diameter of the hair) and therefore a hair set larger, more visible.

*In vivo* studies on the Biot-GHK applied to the lashes are given below in the description.

Topical use is any application on the skin and appendages of a mammal, including humans. This application is performed in a cosmetic. The invention is in particular for preventing or treating loss or thinning of hair or nails caused by aging. The effective amount of GHK peptide according to the invention, that is to say its dosage, depends on the purpose of the topical composition containing it, i.e. cosmetic. It depends on various factors such as the age, the state of the patient, and the mode of administration. An effective amount means a nontoxic amount sufficient to achieve the desired effect.

In a topical composition according to the invention, comprising an effective amount of R₁- Gly-His-Lys-R₂ tripeptide, as defined above, in a physiologically acceptable medium, the GHK peptide, is generally present in proportions of between 0.000001% and 15% by weight based on the total weight of the composition, more preferably from 0.0001% to 5%, depending on the purpose of the composition and the desired effect more or less strong.

All percentages and ratios used herein are by weight of the total composition and all measurements are made at 25°C unless it is otherwise specified.

To improve the appearance means more specially according to the invention, to improve the thickness and the length of hair and thus for eyelashes to improve the overall appearance of the whole set of eyelashes; if eyelashes are better anchored, they will be more numerous, also thicker and longer, and the whole set will be large and visible, which is a very desired effect in cosmetics.

The hair growth cycle is divided into three phases: the anagen phase corresponding to hair growth, the catagen phase corresponding to a halt in growth, and the telogen phase corresponding to a resting phase during which the hair falls out.

The duration of anagen and telogen phases are the key differences between the body and facial hair and the scalp hair: for the body and facial hair the anagen phase is much shorter and the telogen phase much longer.

The peptide of the invention succeeds in improving the anchoring phase of the body and facial hair during the anagen phase, to prevent the loss of hair, then in extending the two following phases, thus extending the time of hair growing and extending the resting time before the fall of the hair. Accordingly, it acts directly on the physiology of the hair.

This is particularly advantageous for the eyelashes. The products used conventionally for the eyelashes, mascara-type cares, work by depositing on the existing lashes a layer of a waxy thickener compound, with the disadvantage of making the eyelashes fragile and brittle and forming mostly unsightly packs. In contrast, the compound of the invention will have a treatment effect, continuously, at the cellular level and moreover independent of makeup.

### DETAILED DESCRIPTION

The expression "physiologically acceptable medium" means according to the present invention, without limitation, an aqueous or hydro-alcoholic solution, a water-in-oil emulsion, an oil-in-water emulsion, a micro-emulsion, an aqueous gel, an anhydrous gel, a serum, a dispersion of vesicles or a powder.

"Physiologically acceptable" means that the compositions are suitable for topical or transdermal use in contact with mucous membranes, nails, scalp, hair, and skin of mammals, and particularly human, compositions that can also be ingested or injected into the skin, without risk of toxicity, incompatibility, instability, allergic response, and others.

"The physiologically acceptable medium" forms what is commonly called the excipient of the composition.

For example, the Biot-GHK can be dissolved in an alcohol-based matrix, for example consisting of a mixture water/glycerin.

The present invention also provides a method of cosmetic treatment to improve the overall condition of eyelashes, comprising applying to the hair said composition to their base (anchor) thereof and/or on the hair themselves. The peptide can thus be proposed for an application to the eyelashes using for example a brush, a liner, a pen or a pencil in the way of an eyeliner or in the way of a mascara using a brush.

The present invention also provides a kit comprising, on the first part, an applicator, such as for example a brush a liner, a pen, a pencil or a ball type "roll on" and, on the second part, the composition of the invention in a compartment. Another compartment may be further adapted for a makeup product to color eyelashes, for example.

The peptide may be combined with other active ingredients at effective concentrations that can act synergistically to achieve the desired effects on the hair described for the invention, such as the following agents:
- Vitamin B complexes, or derivatives, conjugates, homologues or analogs selected from: biotin, pantothenic acid (Vitamin B5), panthenol (2,4-dihydroxy-N-(3-hydroxypropyl)-3,3-dimethylbutanamide), reduced form of pantothenic acid, pantetheine (amidated cysteamine analog of pantothenic acid), pantethine (panthetheine dimer, intermediate in the production of coenzyme A in the body), C1-C30 alkyl esters of pantothenic acid, C1-C30 alkyl esters of the carboxylic acid of panthenol, C1-C30 alkyl ethers of panthenol;
- hyaluronic acid or hyaluronate;
- glucosamine or glycosaminoglycans;
- synthetic analogs of prostaglandins of F-type, preferably the 17-phenyl trinor PGF2α ethyl amide (Bimaprost™ Allergan™);
- inhibitors of 5-alpha reductase, which help to regulate the activity of sebaceous glands and which reduce the level of sebum in the skin, preferably spironolactone, Zinc PCA or oleanolic acid.
Panthenol is preferably combined in the composition of the invention. In cosmetics, panthenol is used, among others, as a moisturizer and humectant, emollient. It lubricates the hair shaft and binds to the hair shaft.

Preferably panthenol (which may be in the D-form as well as in a racemic mixture of D and L forms) is included in the composition at effective concentrations between about 0.001% to 5% by weight, based on the total weight of the composition.

Biotin, also known as vitamin B7, may also be included in the compositions of the present invention, preferably at effective concentrations between about 0.001% to about 10%, between 0.001% and 1%, or 0.05% to 0, 1% by weight based on the total weight of the composition. Biotin can be used to further promote the healthy growth of skin and hair.

Glucosamine and/or glycosaminoglycans may also be included in the compositions of the present invention, preferably at concentrations between 0.1% to 10%, between 0.1% and 8%, 0.5% and 7%, or 1% to 5%, by weight based on the total weight of the composition. The glucosamine and/or glycosaminoglycans are used for their anti-inflammatory properties.

As examples, one can also cite the following products to reinforce or supplement the composition, recommended for hair care: Trychogen™ (marketed by Laboratoires Sérobiologiques/Cognis); Phytokeratin™ (marketed by Arch), RonaCare Biotin Plus™ (Merck Chemicals France), Capigen™, Capilectin™, Capislow™, Procapil™, Procapil™ AF, Hairspa™, Moist24™, Centaurium™ EL, Héliogénol™, Chronodyn™, Norgel™, Osmopur™, Fruitbio™, Esculoside™, Ecodermine™, Ceramide 2™, Ceramide A2™, Ceramide HO3™, Calmosensine™, Biodermine™, Bacocalmine™, Ac.net™, Osmocide™ 4 (marketed by Sederma), Kerasol™, Tritisol™, Crodasone W™ Hydrotiticum PVP™ (marketed by Croda), Double-Lash™ (Malava), shea butter, etc..

The composition according to the present invention can be applied to the eyelashes in any form or vehicles known to the skilled person, in particular in the form of solution, dispersion, emulsion, paste, or powder, individually or as a premix or vehicled individually or as a premix in vectors such as macro-, micro-, or nanocapsules, macro-, micro- or , nanospheres, liposomes, oleosomes or chylomicrons, macro-, micro-, or nanoparticles or macro-, micro or nanosponges, spores or exines, micro or nano emulsions or adsorbed on organic polymer powders, talcs, bentonites, or other inorganic or organic supports.

In cosmetics in particular, uses can be offered for example in ranges of care, makeup-care for eyelashes or as a treatment cream. The peptide according to the present invention may be used in any form whatsoever, in a form bound to or incorporated in or absorbed in or adsorbed on macro-, micro-, and nanoparticles, or macro-, micro-, and nano-capsules, for the treatment of textiles, natural or synthetic fibers, wools, and any materials that may be used for clothing or underwear for day or night intended to come into contact with the skin, handkerchiefs or cloths, to exert their cosmetic effect via this skin/textile contact and to permit continuous topical delivery.

### Additional ingredients

The composition of the invention may contain other ingredients that can enhance the look and feel of hair or fingernail, increase the dressing and flexibility of the hair, make the styling easier, improve brilliance and shine and also improve the hair texture.

The CTFA International cosmetic ingredient dictionary & handbook (13th Ed. 2010) (published by the Cosmetic, Toiletry, and Fragrance Association, Inc., Washington, D.C.) describes a non-limited wide variety of cosmetic and pharmaceutical ingredients conventionally used in the skin care industry that can be used as additional ingredients/compounds in the compositions for the present invention. Examples of these ingredient classes include, but are not limited to: healing agents, skin anti-aging agents, anti-wrinkle agents, anti-atrophy agents, skin moisturizing agents, skin smoothing agents, antibacterial agents, anti-parasitic agents, antifungal agents, fungicidal agents, fungistatic agents, bactericidal agents, bacteriostatic agents, antimicrobial agents, anti-inflammatory agents, anti-pruriginous agents, anesthetic agents, antiviral agents, keratolytic agents, free radicals scavengers, anti-seborrhea agents, antidandruff agents, the agents modulating differentiation, proliferation or pigmentation of the skin and agents accelerating penetration, desquamating agents, melanin synthesis stimulating or inhibiting agents, whitening, depigmenting or lightening agents, pro-pigmenting agents, self-tanning agents, NO-synthase inhibiting agents, antioxidants, free radical scavengers and/or agents against atmospheric pollution, reactive carbonyl species scavengers, anti-glycation agents, tightening agents, agents stimulating the synthesis of dermal or epidermal macromolecules and/or capable of inhibiting or preventing their degradation, such as for example collagen synthesis stimulating agents, elastin synthesis stimulating agents, decorin synthesis stimulating agents, laminin synthesis stimulating agents, defensin synthesis stimulating agents, chaperone synthesis stimulating agents, aquaporin synthesis stimulation agents, hyaluronic acid synthesis stimulating agents, fibronectin synthesis stimulating agents, sirtuin synthesis-stimulating agents, agents stimulating the synthesis of lipids and components of the stratum corneum (ceramides, fatty acids, etc.), collagen degradation inhibiting agents elastin degradation inhibiting agents, agents stimulating fibroblast proliferation, agents stimulating keratinocyte proliferation, adipocyte proliferation stimulating agents, melanocyte proliferation stimulating agents, keratinocyte differentiation stimulating agents, adipocyte differentiation stimulating agents, acetylcholinesterase inhibiting agents, glycosaminoglycan synthesis stimulating agents, DNA repair agents, DNA protecting agents, anti-itching agents, agents for the treatment and/or care of sensitive skin, firming agents, anti-stretch mark agents, astringent agents, sebum production regulating agents, dermo-relaxing agents, healing adjuvant agents, re-epithelialization stimulating agents, re-epithelialization co-adjuvant agents, cytokine growth factors, calming agents, anti-inflammatory agents, agents acting on capillary circulation and/or microcirculation, angiogenesis stimulating agents, vascular permeability inhibiting agents, agents acting on cell metabolism, agents able to improve dermal-epidermal junction, agents inducing hair growth, hair growth inhibiting or retardant agents, muscle relaxants agents, antipollution and/or anti-free radical agents, lipolysis stimulating agents, slimming agents, anti-cellulite agents, agents acting on the microcirculation, agents acting on the energy metabolism of the cells, cleaning agents, hair conditioning agents, hair styling agents, hair growth promoters, sunscreen agents, total sunscreen agents, make-up agents, detergents, pharmaceutical products, emulsifiers, emollients, organic solvents, antiseptic agents, deodorant actives, physiologically acceptable carriers, surfactants, abrasives, absorbents, aesthetic components such as fragrances, pigments, dyes, colorants, natural colorants, essential oils, touch agents, cosmetic astringents, anti-acne agents, anti-coagulation agents, antifoaming agents, antioxidants, binders, biological additives, enzymes, enzymatic inhibitors, enzyme-inducing agents, coenzymes, chelating agents, plant extracts, plant derivatives, essential oils, marine extracts, agents obtained from a bio-fermentation or a biotechnological process, mineral salts, cell extracts, sunscreens (organic or mineral photoprotective agents active against ultraviolet A and/or B rays), ceramides, peptides, buffers, volumizing agents, chelating agents, chemical additives, colorants, cosmetic biocides, denaturants, medical astringents, external analgesics, film formers, such as polymers, for exacerbing film-forming properties and substantivity of the composition, quaternary derivatives, substantivity increasing agents, opacifying agents, pH adjusters and regulators (e.g. triethanolamine), propellants, reducing agents, sequestrants, decoloring and/or lightening agents, skin-conditioning agents (e.g., humectants, including miscellaneous and occlusive), moisture retaining agents, alphahydroxyacids, betahydroxyacids, moisturizers, epidermal hydrolytic enzymes, healing and/or calming agents, skin treating agents, anti-wrinkle agents, agents that reduce or treat bags under the eyes, exfoliating agents, thickeners, softening agents, gelling polymers, vitamins and their derivatives, wetting agents, peeling agents, soothing agents, curative agents of the skin, lignans, preservatives (i.e. phenoxyethanol and parabens), anti UV, cytotoxic agents, anti-neoplastics, viscosity modifiers, non-volatile solvents, pearling agents, anti-perspirant agents, depilatories, vaccine, perfumed water, skin restructuring agent (i.e. Siegesbeckia orientalis extract), excipients, charges, minerals, anti-mycobacterial agents, anti-allergenic agents, H1 or H2 antihistaminics, anti-irritants, agents stimulating the immune system, agents inhibiting the immune system, insect repellents, lubricants, pigments or dyes, hypopigmentation agents, preservatives, light stabilizers, and mixtures thereof, as long as they are physically and chemically compatible with the other ingredients of the composition and especially with the active ingredients of the present invention.

Also the nature of these additional ingredients should not unacceptably alter the benefits of the active ingredients of the invention. These additional ingredients can be synthetic or natural such as plants extracts, or come from a bio-fermentation process. Additional examples can be found in the CTFA Cosmetic Ingredient Handbook.

Such additional active ingredient/compound can be selected from the group consisting of: sugar amines, glucosamine, D-glucosamine, N-acetyl glucosamine, N-acetyl-D-glucosamine, mannosamine, N-acetyl mannosamine, galactosamine, N-acetyl galactosamine, B3 vitamin and its derivatives, niacinamide, sodium dehydroacetate, dehydroacetic acid and its salts, phytosterols, salicylic acid compounds, hexamidines, dialkanoyl hydroxyproline compounds, soy extracts and derivatives, equol, isoflavones, flavonoids, phytantriol, farnesol, geraniol, bisabolol, peptides and their derivatives, di-, tri-, tetra-, penta-, and hexapeptides and their derivatives, KTTKS (SEQ ID NO: 2), Pal-KTTKS (SEQ ID NO: 3), carnosine, N-acyl amino acid compounds, retinoids, retinyl propionate, retinol, retinyl palmitate, retinyl acetate, retinal, retinoic acid, water-soluble vitamins, ascorbates, C vitamin, ascorbyl glucoside, ascorbyl palmitate, magnesium ascorbyl phosphate, sodium ascorbyl phosphate, vitamin B and their salts and derivatives, B1 vitamin, B2 vitamin, B6 vitamin, B12 vitamin, provitamins and their salts and derivatives, K vitamin and derivatives, pantothenic acid and its derivatives, pantothenyl ethyl ether, panthenol and derivatives, dexpanthenol, biotin, amino acids and their salts and derivatives, water soluble amino acids, asparagine, alanine, indole, glutamic acid, water insoluble vitamins, A vitamin, E vitamin, F vitamin, D vitamin and mono-,di-, and tri-terpenoids compounds, beta-ionol, cedrol, and their derivatives, water insoluble amino acids, tyrosine, tryptamine, particulate materials, butylated hydroxytoluene, butylated hydroxyanisole, allantoin, tocopherol nicotinate, tocopherol, tocopherol esters, palmitoyl-Gly-His-Lys (Pal-GHK), phytosterol, hydroxy acids, glycolic acid, lactic acid, lactobionic acid, keto acids, pyruvic acid, phytic acid, lysophosphatidic acid, stilbenes, cinnamates, resveratrol, kinetin, zeatin, dimethylaminoethanol, natural peptides, soy peptides, salts of sugar acids, Mn gluconate, Zn gluconate, piroctone olamine, 3,4,4'-trichlorocarbanilide, triclocarban, Zn pyrithione, hydroquinone, kojic acid, ascorbic acid, magnesium ascorbyl phosphate, ascorbyl glucoside, pyridoxine, aloe vera, terpene alcohols, allantoin, bisabolol, dipotassium glycyrrhizinate, glycerol acid, sorbitol acid, pentaerythritol acid, pyrrolidone acid and salts, dihydroxyacetone, erythrulose, glyceraldehyde, tartaraldehyde, clove oil, menthol, camphor, eucalyptus oil, eugenol, menthyl lactate, witch hazel distillate, eicosene and vinyl pyrrolidone copolymer, iodopropyl butylcarbamate, a polysaccharide, an essential fatty acid, a salicylate, glycyrrhetinic acid, carotenoids, ceramides and pseudo-ceramides, a lipid complex, oils in general of natural origin such shea butter, apricot oil, onagre oil, prune oil, palm oil, monoi oil, hydroquinone, HEPES, procysteine, O-octanoyl-6-D-maltose, the disodium salt of methylglycinediacetic acid, steroids such as diosgenin and derivatives of DHEA, DHEA or dehydroepiandrosterone and/or a precursor or chemical or biological derivative thereof, N-ethyloxycarbonyl-4-para-aminophenol, blueberries extracts, phytohormones, extracts of the yeast Saccharomyces cerevisiae, extracts of algae, extracts of soyabean, lupin, maize and/or peas, alverine and its salts, in particular alverine citrate, extract of butcher's broom and of horse chestnut, and mixtures thereof, a metalloproteinase inhibitor. Further skin care and hair care active ingredients that are particularly useful combined with the composition can be found in SEDERMA commercial literature and on the website www.sederma.fr. The following commercial actives can also be mentioned, as examples: betain, glycerol, Actimoist Bio 2™ (Active organics), AquaCacteen™ (Mibelle AG Cosmetics), Aquaphyline™ (Silab), AquaregulK™ (Solabia), Carciline™ (Greentech), Codiavelane™ (Biotech Marine), Dermaflux™ (Arch Chemicals, Inc), Hydra'Flow™ (Sochibo), Hydromoist L™ (Symrise), RenovHyal™ (Soliance), Seamoss™ (Biotech Marine), Essenskin™ (Sederma), Moist 24™ (Sederma), Argireline™ (trade name of the acetyl hexapeptide-3 of Lipotec), spilanthol or an extract of Acmella oleracea known under the name Gatuline Expression™, an extract of Boswellia serrata known under the name Boswellin™, Deepaline PVB™ (Seppic), Syn-AKE™ (Pentapharm), Ameliox™, Bioxilift™ (Silab), Juvinity™ (Sederma), Revidrat™ (Sederma), Chronodynt™ (Sederma), or mixtures thereof.

Among other plant extracts which can be combined with the composition of the invention, there may more particularly be mentioned extracts of Ivy, in particular English Ivy (*Hedera Helix*), of *Bupleurum chinensis,* of *Bupleurum Falcatum,* of arnica (*Arnica Montana L*)*,* of rosemary (*Rosmarinus officinalis N*)*,* of marigold (*Calendula officinalis*)*,* of sage (*Salvia officinalis L*)*,* of ginseng (*Panax ginseng*), of ginko biloba, of St.-John's-Wort (*Hyperycum Perforatum*)*,* of butcher's-broom (*Ruscus aculeatus L*)*,* of European meadowsweet (*Filipendula ulmaria L*)*,* of big- flowered Jarva tea (*Orthosiphon Stamincus Benth*)*,* of algae (*Fucus Vesiculosus*), of birch (*Betula alba*), of green tea, of cola nuts (*Cola Nipida*), of horse-chestnut, of bamboo, of *Centella asiatica*, of heather, of fucus, of willow, of mouse-ear, of escine, of cangzhu, of *chrysanthellum indicum,* of the plants of the *Armeniacea* genus, *Atractylodis Platicodon, Sinnomenum, Pharbitidis, Flemingia,* of *Coleus* such as C. *Forskohlii, C. blumei, C. esquirolii, C. scutellaroides, C. xanthantus* and *C*. *Barbatus,* such as the extract of root of *Coleus barbatus,* extracts of *Ballote*, of *Guioa,* of *Davallia*, of *Terminalia,* of *Barringtonia,* of *Trema*, of *antirobia*, *cecropia*, *argania*, *dioscoreae* such as *Dioscorea opposita* or Mexican, extracts of *Ammi visnaga*, of *Siegesbeckia,* in particular *Siegesbeckia orientalis,* vegetable extracts of the family of *Ericaceae,* in particular bilberry extracts (*Vaccinium angustifollium*) or *Arctostaphylos uva ursi*, *aloe vera*, plant containing sterols (e.g., phytosterol), Manjistha (extracted from plants of the genus *Rubia*, particularly *Rubia Cordifolia*), and Guggal (extracted from plants of the genus *Commiphora*, particularly *Commiphora Mukul*), kola extract, chamomile, red clover extract, Piper methysticum extract (Kava Kava™ from SEDERMA), *Bacopa monieri* extract (Bacocalmine™ from SEDERMA) and sea whip extract, extracts of *Glycyrrhiza glabra*, of mulberry, of *melaleuca* (tea tree), of *Larrea divaricata*, of *Rabdosia rubescens,* of *Euglena gracilis,* of *Fibraurea recisa Hirudinea*, of *Chaparral Sorghum,* of sun flower extract, of *Enantia chlorantha,* of Mitracarpe of *Spermacocea* genus, of *Buchu barosma*, of *Lawsonia inermis L.,* of *Adiantium Capillus-Veneris L.,* of *Chelidonium majus,* of *Luffa cylindrica*, of Japanese Mandarin (*Citrus reticulata Blanco* var. *unshiu*), of *Camelia sinensis*, of *Imperata cylindrica*, of *Glaucium Flavum*, of *Cupressus Sempervirens*, of *Polygonatum multiflorum*, of *loveyly hemsleya*, of *Sambucus Nigra*, of *Phaseolus lunatus*, of *Centaurium,* of *Macrocystis Pyrifera*, of *Turnera Diffusa*, of *Anemarrhena asphodeloides*, of *Portulaca pilosa*, of *Humulus lupulus,* of *Coffea Arabica,* of *Ilex Paraguariensis,* or of *Zingimber Zerumbet Smith.*

The compositions of the present invention may include peptides, including, without limitation, the di-, tri-, tetra-, penta-and hexapeptides and their derivatives. According to a particular embodiment, the concentration of the additional peptide, in the composition, ranges from 1x10⁻⁷% and 20%, preferably from 1x10⁻⁶% and 10%, preferably between 1x10⁻⁵% and 5% by weight.

According to the present invention, the term "peptide" refers to peptides containing 10 amino acids or less, their derivatives, and isomers. The term "peptides" refers to both natural peptides and synthetic peptides. It also refers to compositions that contain peptides which are found in nature, and/or are commercially available.

Suitable dipeptides for use herein include but are not limited to carnosine (beta-AH), YR, VW, NF, DF, KT, KC, CK, KP, KK or TT. Suitable tripeptides for use herein include, but are not limited to RKR, HGG, GHK, GKH, GGH, GHG, KFK, KPK, KMOK, KMO₂K or KAvaK. Suitable tetrapeptides for use herein include but are not limited to RSRK (SEQ ID NO: 4), GQPR (SEQ ID NO: 5) or KTFK (SEQ ID NO: 6). Suitable pentapeptides include, but are not limited to KTTKS (SEQ ID NO: 2). Suitable hexapeptides include but are not limited to GKTTKS (SEQ ID NO: 7), VGVAPG (SEQ ID NO: 8).

Other suitable peptides for use herein include, but are not limited to lipophilic derivatives of peptides, preferably palmitoyl derivatives, and metal complexes as aforementioned (e.g. copper complex of the tripeptide HGG). Preferred dipeptide derivatives include N-Palmitoyl-beta-Ala-His, N-Acetyl-Tyr-Arg-hexadecylester (Calmosensine™, Idealift™ from Sederma). Preferred tripeptide derivatives include N-Palmitoyl-Gly-Lys-His, (Pal-GKH from Sederma), the copper derivative of HGG (Lamin™ from Sigma), Lipospondin (N-Elaidoyl-KFK) and its analogs of conservative substitution, N-Acetyl-RKR-NH2 (Peptide CK+), N-Biot-GHK (from Sederma), Pal-KMO2K (Sederma) and derivatives thereof. Suitable tetrapeptide derivatives for use according to the present invention include, but are not limited to, N-palmitoyl-GQPR (SEQ ID NO: 1) (from Sederma), suitable pentapeptide derivatives for use herein include, but are not limited to, N-Palmitoyl-KTTKS (SEQ ID NO: 3) (available as Matrixyl™ from Sederma), N-Palmitoyl-Tyr-Gly-Gly-Phe-X (SEQ ID NO: 9) with X Met or Leu or mixtures thereof. Suitable hexapeptide derivatives for use herein include, but are not limited to, N-Palmitoyl-VGVAPG (SEQ ID NO: 10) and derivatives thereof. The mixture of Pal-GHK and Pal-GQPR (SEQ ID NO: 1) (Matrixyl™ 3000, Sederma) can also be mentioned.

The preferred compositions commercially available containing a tripeptide or a derivative include Biopeptide-CL™, Maxilip™, Biobustyl™ and Matrixyl™synthe'6™ of Sederma. The compositions commercially available preferred sources of tetrapeptides include Rigin™, Eyeliss™, Matrixyl™ Reloaded and Matrixyl 3000™ which contain between 50 and 500 ppm of Palmitoyl-GQPR (SEQ ID NO: 1) and carrier, proposed by Sederma.

The following marketed peptides can be mentioned as well as additional active ingredients: Vialox™, Syn-ake™ or Syn-Coll™ (Pentapharm), Hydroxyprolisilane CN™ (Exsymol), Argireline™, Leuphasyl™, Aldenine™, Trylgen™, Eyeseryl™, Serilesine™ or Decorinyl™ (Lipotec), Collaxyl™ or Quintescine™ (Vincience), BONT-L-Peptide™ (lnfinitec Activos), Cytokinol™LS (Laboratoires Serobiologiques/Cognis), Kollaren™, IP2000™ or Meliprene™ (Institut Européen de Biologie Cellulaire), Neutrazen™ (Innovations), ECM-Protect™ (Atrium Innovations), Timp-Peptide™ or ECM Moduline™ (lnfinitec Activos).

### Cosmetic treatment method

The present invention also concerns a cosmetic treatment method to improve the general condition of the hair that is eyelashes, comprising the topical application to the hair of an effective amount of a composition according to the invention as recited above.

The composition according to the invention may be applied locally onto targeted areas. One of the major advantages of the present invention resides in the ability whenever necessary or desirable to be able to apply local selective "gentle" treatments through this topical, non-invasive method of application. The application can be carried out very locally using a liner, a pencil, a roll-on applicator, a brush, a micro-cannula, applicators which are well known to the skilled person.

According to other specific features, the topical cosmetic treatment method according to the invention can be combined with one or several other treatment methods targeting the skin such as luminotherapy, heat or aromatherapy treatments.

According to the invention, devices with several compartments or kits may be proposed to apply the method described above which may include for example and non-restrictively, a first compartment containing a composition comprising the peptide of the invention, and in a second compartment a composition containing another active ingredient and/or excipient, the compositions contained in the said first and second compartments in this case being considered to be a combination composition for simultaneous, separate or stepwise use in time, particularly in one of the treatment methods recited above.

The treatment method of the invention is particularly suited for cosmetic treatment of the eyelashes including:
- improve the anchoring and/or
- make grow, increase the length and/or
- increase the thickness and/or volume and/or
- increase flexibility and/or
- increase the protection and/or
- increase the strength.

### GALENIC

Different examples of formulas (mascara, lotion, serum, etc..) are given below containing the GHK peptide according to the invention as active ingredient. These formulas can also incorporate additional active ingredients, the latter coming if appropriate to support and/or in addition to the activity of the active ingredient of the invention. These ingredients can be of any class according to their(s) function(s), the application site (body, face, hands, etc.), the desired final effect and the target consumer.

### Example 1: Volumizing Black Mascara

| **US INCI Name (Ingredients)** | **EU INCI Name** | |
|---|---|---|
| Water | Aqua | + |
| Beeswax | Cera Alba | + |
| Acacia Senegal Gum Extract | Acacia Senegal | + |
| Stearic Acid | Stearic Acid | + |
| Massocare OPC A2 (Styrene/Acrylates Copolymer) | Styrene/Acrylates Copolymer | + |
| Glycerin | Glycerin | + |
| PVP | PVP | + |
| Ingredient according to the invention comprising 330 ppm of Biot-GHK in a hydroalcoholic matrix (water/glycerin) | Biotinoyl tripeptide-1 | 2.00% |
| Aminomethyl Propanediol | Aminomethyl Propanediol | + |
| Triethanolamine 99 % | Triethanolamine | + |
| Chlorphenesin | Chlorphenesin | + |
| Methylisothiazolinone | Methylisothiazolinone | + |
| Iron Oxides | CI 77499 | + |

### Example of additional ingredient that can be added to this formula:

- D-Panthenol to strengthen the volumizing effect and confer a protective moisturizer effect: 100ppm added simultaneously with the peptide.

### Example 2: Makeup Remover Lotion

| **Ingredient** | **INCI Name** | % |
|---|---|---|
| **Phase A** | | |
| H₂O | Water | Qsp100 |
| Glycerin | Glycerin | 30.00 |
| Potassium Sorbate | Potassium Sorbate | 0.10 |

| **Phase B** | | |
|---|---|---|
| Butylene Glycol | Butylene Glycol | 35.00 |
| Synperonic PE/F 127 | Poloxamer 407 | 1.00 |

| **Phase C** | | |
|---|---|---|
| Crodateric CAB 30-LQ | Cocamidopropyl Betaine | 1.00 |

| **Phase D** | | |
|---|---|---|
| Tween 20 | Polysorbate-20 | 2.00 |
| Preservative | | qs |
| Parfum Douceur Bleuet | Fragrance | 0.10 |

| **Phase E** | | |
|---|---|---|
| Ingredient according to the invention comprising 330 ppm of Biot-GHK in a hydroalcoholic matrix (water/glycerin) | Biotinoyl tripeptide-1 | 2.00 |

### Procedure:

Weigh and homogenize phase A and then heat in a water bath at 75°C. Weigh and heat phase B in a water bath at 75°C. Add phase B to la phase A under rapid stirring. Weigh and add phase C in phase A+B under gentle stirring at 35°C. Weigh and homogenize phase D. Add phase D in phase A+B+C under gentle stirring. Weigh and add phase E in the previous phase, homogenize well. Check pH.

### Examples of additional ingredients that can be added to this formula:

- D-Panthenol to strengthen the volumizing effect and confer a protective moisturizer effect: 100ppm added simultaneously with the peptide in phase E.
- Eyeliss™: an active ingredient sold by SEDERMA (WO2003/068141), which helps prevent and fight against the appearance of bags under the eyes. 2% of this ingredient may for example be added to phase E of the formula.
- Haloxyl™: active ingredient marketed by SEDERMA (WO2005/102266), which improves the eye contour by reducing dark circles. 3% of this ingredient may for example be added to phase E of the formula simultaneously with the ingredient of the invention.
- Bimaprost™: active ingredient wich enhance the eyelashes growth, marketed by Allergan ; 0.01 % of this ingredient may for example be added to phase E of the formula.
- Chronodyn™: active ingredient (containing Euglena extract (*Euglena gracilis*) which tones and firms the skin, and erases visible signs of fatigue marketed by SEDERMA (WO2006/075311). 3% of this ingredient may for example be added to the phase E.

### Example 3: Mascara

| **Ingredient** | **Nom INCI** | % |
|---|---|---|
| **Phase A** | | |
| H₂O | Water | qsp |
| Chlorphenesin | Chlorphenesin | 0.10 |
| Glycerin | Glycerin | 2.00 |

| **Phase B** | | |
|---|---|---|
| AMPD Ultra PC | Aminomethyl Propanediol | 0.90 |
| PVP K 30 | PVP | 2.00 |
| Kahlgum 6644 | Acacia Senegal Gum | 10.00 |

| **Phase C** | | |
|---|---|---|
| Iron oxides | CI 77499 | 10.00 |

| **Phase D** | | |
|---|---|---|
| Cerabeil Blanche | Beeswax | 10.00 |
| Stearic Acid | Stearic Acid | 10.00 |

| **Phase E** | | |
|---|---|---|
| H₂O | Water | 10.00 |
| TEA 99% | Triethanolamine | 2.00 |

| **Phase F** | | |
|---|---|---|
| Ingredient according to the invention comprising 330 ppm of Biot-GHK in a hydroalcoholic matrix (water/glycerin) | Biotinoyl tripeptide-1 | 2.00 |

| **Phase G** | | |
|---|---|---|
| Isothiazolinone | Methylchloroisothiazolinone & Methylisothiazolinone | 0.05 |

| **Phase H** | | |
|---|---|---|
| Massocare OPC A2 | Styrene & Acrylates Copolymer | 6.00 |

### Procedure:

Weigh phase A, homogenize with helix stirring. Pour phase B, under helix stirring, during 1 hour. Weigh and add phase C in phase A+B under stirring. Heat phase A+B+C in a water bath at 85°C. Weigh phase D, and then heat it in a water bath at 85°C. Weigh and homogenize phase E. Add phase D in phase A+B+C under stirring. Extemporaneously, add phase E in phase A+B+C+D. Then stir the emulsion under ultra-strong agitation during 2 minutes. Add phase F at around 65°C in the previous phase under stirring. Weigh and add phase G, at under 40°C. Weigh and add phase H, well homogenize. Check pH.

### Examples of additional ingredients that can be added to this formula:

- D-Panthenol to strengthen the volumizing effect and confer a protective moisturizer effect: 100ppm added simultaneously with the peptide in phase F.
- Ceramide 2™: active ingredient anti-ageing marketed by Sederma (WO 2004/101609) Having a restructuring function and that keep the hydratation and the integrity of the skin barrier; 0.05% can be added into phase D.
- Ceramide HO3™: active ingredient which reinforces the skin barrier, marketed by Sederma; 0.05% can be added into phase D.
- Ceramide A2™: active ingredient marketed by Sederma (FR2738483) which reinforces the hair structure, the protection and the sheathing of damaged hair; 1% can be added to phase F.
- Capislow™: active ingredient moderator of hair regrowth, comprising *Larrea divaricata*, marketed by Sederma (WO 2000/28959).
- Procapil™: active ingredient marketed by Sederma (WO 2000/58347). Combination of 3 complementary actived in solution: biot-GHK, apigenin (a flavonoid extracted from citrus) and oleanolic acid (extracted from roots of *Loveyly Hemsleya*) which is used to fight against the hair aging process to prevent the hair loss; 3% can be added to phase F.
- Procapil™AF: Procapil™ without apigenin.
- Capigen™: active ingredient for hair marketed by Sederma (Homotaurin, bacterial filtrate rich in peptides, extracted from sulfomucopolysaccharides of marine origin).
- Capilectine™: active ingredient for hair marketed by Sederma (glycoprotein coming from *Solanum tuberosum L.*)*.*

### Example 4: Mascara

| **Ingredient** | **INCI Name** | % |
|---|---|---|
| **Phase A** | | |
| H₂O | Water | qsp |
| Chlorphenesin | Chlorphenesin | 0.10 |
| Glycerin | Glycerin | 2.00 |

| **Phase B** | | |
|---|---|---|
| AMPD Ultra PC | Aminomethyl Propanediol | 0.90 |
| PVP K 30 | PVP | 2.00 |
| Kahlgum 6644 | Acacia Senegal Gum | 10.00 |

| **Phase C** | | |
|---|---|---|
| Iron Oxides | CI 77499 | 10.00 |

| **Phase D** | | |
|---|---|---|
| Cerabeil Blanche | Beeswax | 10.00 |
| Stearic Acid | Stearic Acid | 10.00 |

| **Phase E** | | |
|---|---|---|
| H₂O | Water | 10.00 |
| TEA 99% | Triethanolamine | 2.00 |

| **Phase F** | | |
|---|---|---|
| Ingredient according to the invention comprising 330 ppm of Biot-GHK in a hydroalcoholic matrix (water/glycerin) | Biotinoyl tripeptide-1 | 2.00 |

| **Phase G** | | |
|---|---|---|
| Isothiazolinone | Methylchloroisothiazolinone & Methylisothiazolinone | 0.05 |

| **Phase H** | | |
|---|---|---|
| Massocare OPC A2 | Stirene & Acrylates Copolymer | 6.00 |

### Procedure:

Weigh phase A, homogenize with stirring. Pour phase B, under helix stirring, during 1 hour. Weigh and add phase C in phase A+B. Heat phase A+B+C in a water bath at 85°C. Weigh phase D, and then heat it in a water bath at 85°C. Weigh and homogenize phase E. Add phase D in phase A+B+C under strong stirring. Extemporaneously, add phase E in phase A+B+C+D. Then stir the emulsion under ultra-strong agitation during 2 minutes. Add phase F at around 65°C in the previous phase under stirring. Weigh and add phase G, at less than 40°C. Weigh and add phase H, well homogenize. Check pH.

### Examples of additional ingredients that can be added to this formula:

- D-Panthenol to strengthen the volumizing effect and confer a protective moisturizer effect: 100ppm added simultaneously with the peptide in phase F.
- Renovage™: an overall anti-ageing active ingredient marketed by Sederma (WO 2006/120646); 3% of this ingredient may for example be added to phase D of the formula.
- Juvinity™: an overall anti-ageing active ingredient marketed by Sederma (WO 2011/125039); 2% of this ingredient may for example be added to phase D of the formula.
- Revidrat™: active ingredient which improves the cohesion of the epidermis and its hydration, marketed by Sederma (WO 2011/086532); 3% of this ingredient may for example be added to phase D of the formula.
- Héliogenol™: antioxidant active ingredient marketed by Sederma (WO1991/11169); 2% of this ingredient may for example be added to phase F of the formula.

### Example 5: « Primer » Mascara (care, pre-mascara or mascara base)

| **Ingredient** | **INCI Name** | % |
|---|---|---|
| **Phase A** | | |
| H₂O | Water | 40.00 |
| Optasense G83 | Carbomer | 0.30 |

| **Phase B** | | |
|---|---|---|
| H₂O | Water | Qsp100 |
| PVP K30 | PVP | 2.00 |

| **Phase C** | | |
|---|---|---|
| Potassium Sorbate | Potassium Sorbate | 0.10 |

| **Phase D** | | |
|---|---|---|
| H₂O | Water | 3.50 |
| NaOH 30% | Sodium Hydroxide | 0.35 |

| **Phase E** | | |
|---|---|---|
| Butylene Glycol | Butylene Glycol | 3.00 |
| Preservative | Preservative | qs |
| Natrosol 250M | Hydroxyethyl Cellulose | 0.75 |

| **Phase F** | | |
|---|---|---|
| Alcohol | Ethanol | 5.00 |

| **Phase G** | | |
|---|---|---|
| Ingredient according to the invention comprising 330 ppm of Biot-GHK in a hydroalcoholic matrix (water/glycerin) | Biotinoyl tripeptide-1 | 2.00 |

| **Phase H** | | |
|---|---|---|
| Parfum douceur de Bleuet | Fragrance | 0.10 |

**Procedure:** Phase A: pour the carbomer into water and let it swell for 1 hour without strirring. Phase B: Pour the PVP K30 into water with rapid stirring, let homogenize 30 minutes. Add phase C in phase A under stirring. Add phase D in phase A+C under stirring. Weigh and homogenize phase E. Add phase E in phase B under stirring. Let swell for 30 minutes. Add phase B+E in A+C+D while stirring. Add phase F, well homogenize. Check pH at 6.40. Add phase G, well homogenize. Add phase H, well homogenize.

### Examples of additional ingredients that can be added to this formula:

- D-Panthenol to strengthen the volumizing effect and confer a protective moisturizer effect: 100ppm added simultaneously with the peptide in phase G.
- Hairspa™: active ingredient marketed by Sederma (WO 2004/004345) which soothes and moisturizes the scalp; 2% of this ingredient may for example be added to phase G of the formula.
- Heliogenol™: antioxidant active ingredient marketed by Sederma (WO1991/11169); 2% of this ingredient may for example be added to phase G of the formula.

### Example 6: Thickener of eyebrows or mustache

| **Ingredient** | **INCI Name** | % |
|---|---|---|
| **Phase A** | | |
| H₂O | Water | 40.00 |
| Optasense G83 | Carbomer | 0.30 |

| **Phase B** | | |
|---|---|---|
| H₂O | Water | Qsp100 |
| PVP K30 | PVP | 2.00 |

| **Phase C** | | |
|---|---|---|
| Potassium Sorbate | Potassium Sorbate | 0.10 |

| **Phase D** | | |
|---|---|---|
| H₂O | Water | 3.50 |
| NaOH 30% | Sodium Hydroxide | 0.35 |

| **Phase E** | | |
|---|---|---|
| Butylene Glycol | Butylene Glycol | 3.00 |
| Preservative | Preservative | qs |
| Natrosol 250M | Hydroxyethyl Cellulose | 0.75 |

| **Phase F** | | |
|---|---|---|
| Pigment/mica | | 5.00 |

| **Phase G** | | |
|---|---|---|
| Ingredient according to the invention comprising 330 ppm of Biot-GHK in a hydroalcoholic matrix (water/glycerin) | Biotinoyl tripeptide-1 | 2.00 |

| **Phase H** | | |
|---|---|---|
| Parfum douceur de Bleuet | Fragrance | 0.10 |

**Procedure:** Phase A: pour the carbomer into water and let it swell for 1 hour without strirring. Phase B: Pour the PVP K30 into water with rapid helix stirring, let homogenize 30 minutes. Add phase C in phase A under helix stirring. Add phase D in phase A+C under blade stirring. Weigh and homogenize phase E. Add phase E in phase B under helix stirring. Let swell for 30 minutes. Add phase B+E in A+C+D while blade stirring. Add phase F, well homogenize. Check pH at 6.40. Add phase G, well homogenize. Add phase H, well homogenize.

### Examples of additional ingredients that can be added to this formula:

- D-Panthenol to strengthen the volumizing effect and confer a protective moisturizer effect: 100ppm added simultaneously with the peptide in phase G.
- Hairspa™: active ingredient marketed by Sederma (WO 2004/004345) which soothes and moisturizes the scalp; 2% of this ingredient may for example be added to phase G of the formula.
- Héliogenol™: antioxidant active ingredient marketed by Sederma (WO1991/11169); 1% of this ingredient may for example be added to phase G of the formula.

According to the invention the combination « GHK peptide and panthenol » is a preferred association. The 2 active compounds may be combined in a single ingredient, the Biot-GHK peptide at 0.033% (330ppm) and the D-panthenol at 0.5% by weight (5000ppm) relative to the total weight of the composition of the ingredient, and the two actives can be added simultaneously to the formula.

### Example 7: Fluid cream for treating nails

| **Ingredient** | **INCI Name** | % |
|---|---|---|
| **Phase A** | | |
| H₂O | Water | Qsp100 |
| Sorbate | Potassium Sorbate | 0.10 |
| MgSO4 | Magnesium Sulfate | 0.70 |

| **Phase B** | | |
|---|---|---|
| ABIL EM 90 | Cetyl PEG/ PPG-10/1 Dimethicone | 3.00 |
| Phenoxyethanol | Phenoxyethanol | qs |
| Syncrowax HRC | Tribehenin | 1.00 |
| Crodamol STS | PPG-3 Benzyl Ether Myristate | 2.00 |
| Parleam Oil | Hydrogenated polyisobutene | 19.00 |

| **Phase C** | | |
|---|---|---|
| Ingredient according to the invention comprising 330 ppm of Biot-GHK in a hydroalcoholic matrix (water/glycerin) | Biotinoyl tripeptide-1 | 2.00 |

| **Phase D** | | |
|---|---|---|
| Perfume | Fragrance | 0.10 |

**Procedure:** Weigh phase A, heat. Mix phase B, heat, mix well. Extemporaneously, add phase C in phase B, homogenize well. Slowly add phase A to phase B+C with stirring. Then add phase D. Stir until cooled.

### Examples of additional ingredients that can be added to this formula:

- D-Panthenol to confer a protective moisturizer effect: 100ppm added simultaneously with the peptide in phase C.

### In vivo STUDIES

### Principle and purpose of the study:

The purpose of the clinical study was to evaluate the following among volunteers:
- lash lengthening,
- lash thickening,
- reinforcement of lash anchorage (decrease in lash loss during mascara removal).

Lashes were photographed in a precise manner that could be reproduced and at a special angle so that lashes could be viewed perfectly. An image analysis was then performed on these photographs to quantify lash length and diameter in the central segment of the lids of female volunteers. Lash volume was inferred from this data.

Concurrent to this quantitative metrological study, two other evaluations were performed: one clinical evaluation in which trained experts scored photos and another evaluation in which the volunteers themselves evaluated the perceived effects using a multi-item questionnaire.

### Protocol

This was a double-blind study that employed non-invasive methods. 30 female volunteers used two mascaras: 1) VOLUMISING BLACK MASCARA (according to Example 1) containing 0.00066% of Biot-GHK and 0.01% of panthenol on one eye and 2) its placebo on the other eye (same formula without the active ingredients of the invention).

The measurements were performed before application (TO) then after 15 and 30 days of daily application. Application during the eight hours preceding a measurement day was not allowed.

The volunteers had to apply each mascara as follows: 3 brush strokes to the outside lashes, 4 brush strokes to the central lashes and 3 brush strokes to the interior lashes. The investigator randomised the products to the right and left eye.

A dermatologist observed and supervised the usual inclusion (criteria, informed consent) and exclusion conditions.

Neither the investigators nor the volunteers reported any intolerance problems.

### Lash length and diameter evaluation:

### Photo measurements

Once the head was correctly positioned on an appropriate mechanism, the lashes were frontally photographed using a Reflex Nikon D300 (12.3 megapixel) digital camera with a "macro" (60 mm F/2.8 GED) lens and a flash. The images were analysed with special software. The calibrated lash images helped determine lash length in mm. The portion of the lids with the lashes was divided into 5 segments, which were numbered from S1 to S5. The lengths and diameters were measured on the lashes of segments S2, S3 and S4 (5 lashes per segment) on each side.

### Self-evaluation by volunteers:

Several self-evaluation questions were posed to the volunteers after 30 days:
1) "After mascara use, my lashes are..."
   a. Longer
   b. Thicker
   c. Better nourished
   d. Strengthened
      With each statement, the volunteers needed to state:
      Totally agree; Agree; Disagree; Totally disagree.
2) "In general, how would you rate the lash lengthening effect"?
   Excellent; Very good; Good; Fair; Poor.
3) "In general, how would you rate the lash thickening effect?"
   Excellent; Very good; Good; Fair; Poor.
4) Regarding the strengthening/anchorage effect: "The product helps prevent lash breakage/loss?"
   Totally agree; Agree; Disagree; Totally disagree

### Strengthening effect evaluation:

In order to evaluate the strengthening effect, two methods were used:

The volunteers were invited to perform a daily rating of the number of lashes lost on each eye after makeup removal.

Moreover, at the end of the study, volunteers were asked to answer the question "The product helps prevent breakage/losses" (of lashes) using the scale:
Totally agree; Agree; Disagree; Totally disagree

### Results

### Measurement of lengthening increase:

**Table 1: Variation in lash length after application of the mascara according to the invention containing the Biot-GHK peptide (in mm, mean values of 30 volunteers).**

| | Mascara of the invention | | | Mascara placebo | | |
|---|---|---|---|---|---|---|
| | T0 | T15 days | T30 days | T0 | T15 days | T30 days |
| Mean (mm) ± SEM | 6.051 ± 0.126 | 6.690 ± 0.124 | 7.081 ± 0.126 | 6.047 ± 0.133 | 6.296 ± 0.131 | 6.433 ± 0.131 |
| Difference TXj - T0 → Max | - | 0.639 1.796 | 1.030 2.108 | - | 0.249 0.559 | 0.387 0.984 |
| Variation (%) Significance → Max | | +10.56% p<0.01 +32% | +17.02% p<0.01 +43% | | +4.12% p<0.01 | +6.38% p<0.01 |
| Increase Significance *versus* placebo | | X 2.6 p<0.01 | X 2.7 p<0.01 | | | |

The measurement of the increase in lash length shows that the mean length of the placebo lashes increased significantly by 4% and 6% at 15 days and 30 days respectively. This effect can be explained by natural lash growth.

At the same time, the mascara according to the invention stimulated natural lash growth, the lash length thus being enhanced by 10% and 17% at 15 days and 30 days respectively. Thanks to the biotinyl-GHK peptide, which stimulates keratinocyte proliferation in the hair bulb, the lashes grow 2.6 times more than the mascara placebo at both study evaluation dates. This difference in growth obtained with the two mascaras is significant and in favor of the mascara of the invention (p<0.01)

### Measurement of lash diameter increase - Photo measurement

The device used to measure the length of the lashes was also used to measure their diameter. The study of the variation of the lash diameter shows that with the placebo, their mean diameter increases by 4% and 7% at 15 and 30 days respectively. (Table 2 below). In parallel, the mascara according to the invention increased the lash diameter by 12% and 19% at 15 and 30 days respectively.

The mascara according to the invention thus increases by a factor of 2.9, in comparison with placebo, the diameter of the lashes after two study periods. This difference between both mascaras is significant and in favor of the mascara according to the invention (p <0.01).

**Table 2: Variation in lash diameter after applying the mascara according to the invention (in µm, mean values of 30 volunteers)**

| | Mascara of the invention | | | Mascara placebo | | |
|---|---|---|---|---|---|---|
| | T0 | T15 days | T30 days | T0 | T15 days | T30 days |
| Mean (µm) ±SEM | 76.1 ± 2.0 | 85.2 ± 2.6 | 90.3 ± 2.8 | 76.9 ± 2.0 | 79.9 ± 2.2 | 82.3 ± 2.3 |
| Difference TXj - T0 | - | 9.0 | 14.2 | - | 3.1 | 5.4 |
| Variation (%) | | +11.96% | +18.66% | | +4.03% | +7.02% |
| Significance | | p<0.01 | p<0.01 | | p<0.01 | p<0.01 |
| → Max | | +33% | +40% | | | |
| Increase | | | | | | |
| Significance | | X 2.9 | X 2.6 | | | |
| *versus* placebo | | p<0.01 | p<0.01 | | | |
| → Max | | 25.3 | 29.9 | | 12.3 | 12.6 |

### Lash growth - Expert clinical evaluation

The evaluation by the 3 experts, analyzing the photos of the lashes of the 30 volunteers, helped demonstrate that the placebo mascara was not perceived as a mascara that promotes lash growth (80% negative opinions); concurrently, for the experts, 86% of the volunteers found that the mascara according to the invention had a moderate to clear effect (43% and 43%; Table 3, below).

**Table 3: Blind evaluation performed by 3 trained experts on lash lengthening in photos of 30 volunteers.**

| | | | | |
|---|---|---|---|---|
| No effect | Placebo | | | 80% |
| | Of the invention | | | 13% |
| | | | | |
| Moderate effect | Placebo | | | 13% |
| | Of the invention | | | 43% |
| | | | | |
| Clear effect | Placebo | | | 7% |
| | Of the invention | | | 43% |
| | | | | |
| % opinion | 0 | 50% | | 100% |

### Lash thickening increase - Expert clinical evaluation

Furthermore, the evaluation by the 3 experts of the lash thickening of the 30 volunteers, helped demonstrate that the placebo mascara was not perceived as a mascara that promotes an increase in lash diameter (83% negative opinions); concurrently, 74% of the volunteers found that the mascara according to the invention had a moderate to clear effect (27% and 47%; Table 4, below).

**Table 4: Blind evaluation performed by 3 trained experts on increased lash diameter in photos of 30 volunteers**

| | | | | |
|---|---|---|---|---|
| No effect | Placebo | | | 83% |
| | Of the invention | | | 26% |
| | | | | |
| Moderate effect | Placebo | | | 17% |
| | Of the invention | | | 27% |
| | | | | |
| Clear effect | Placebo | | | 0% |
| | Of the invention | | | 47% |
| | | | | |
| % opinion | 0 | 50% | | 100% |

### Lash anchorage - Self-evaluation

In order to evaluate the strengthening effect, the volunteers were invited to perform a daily rating of the number of lashes lost on each eye after makeup removal. This count confirms the improvement in the lash dressing and suggests the strengthening of the anchorage in the infundibulum.

A study has shown that the use of the mascara according to the invention reduces the loss of lashes during makeup removal. The mean loss reduction was 4 lashes (at 15 days) and 9 lashes (at 30 days) compared with the placebo mascara.

This measure is complemented by the self-evaluation questionnaire regarding to the prevention of lash breakage and loss. The 30 volunteers were of like mind on the mascara according to the invention: 70% agreed to strongly agreed on this point.

The 30 volunteers rated their own perceived effects following the mascara use.

Regarding the mascara according to the invention, eyelashes appear longer ("Agree" + "Totally agree": 70%); thicker ("Agree" + "Totally agree": 73%); nourished (80%) and strengthened ("Agree" + "Totally agree": 57+13= 70%).

In parallel, the volunteers answered three questions about the intensity of perceived effects. On the length of eyelashes 73% of the opinions agreed to describe as good to excellent.

On the thickening, 74% of the opinions agreed to describe as good to excellent.

This study clearly shows that after a one month eyelash treatment using a mascara according to the invention leads to a significant increase in lash length *(p< 0.01)* of about 17% which accords with the clinical evaluation by the trained experts who confirm a visible to very visible effect on 87% of the panel.

In parallel, the lashes treated with the placebo mascara did not grow as much as the ones with the mascara according to the invention, making them unnoticeable to the naked eye (no effect 80%).

A great majority of the volunteers noticed that their lashes were longer (70%) and the intensity of the effect was good to very good (73% opinions).

This study also showed, with complementary methods, that lashes were thicker thanks to the use of the mascara according to the invention (+19%) against placebo (7%). A significant difference *(p<0.01).*

The trained experts were able to notice this improvement (noted in 74% of the cases, the placebo showing no effect in 83% of the cases). Finally, the volunteers indicated that their lashes were thicker for 73% of them, an overall phenomenon being good to excellent in 73% of the cases.

This data permitted to calculate the lash volume which clearly increases in just 15 days with the mascara of the invention (+40%) and even more after 30 days (67%). The observations made by the experts and the volunteers are thus confirmed.

The fortifying effect of the mascara according to the invention was appreciated by a regular counting of lashes, the number of lashes lost during make-up removal were less important with the mascara of the invention than with the placebo (mean difference of 4 lashes (15 days) to 9 lashes (30 days)). 70% of the volunteers noted that the mascara according to the invention strengthened their lashes, and helped to prevent lash loss and breakage.

The same volunteers noted that the mascara according to the invention nourished the lashes (80% opinions).

These *in vivo* studies show that the mascara according to the invention improves the visible appearance of the lashed (length, thickness, volume) and their anchorage in the eyelid. Added to a basic mascara formula at a concentration of 2%, the Biot-GHK peptide leads to an increase in the length and diameter of eyelashes after 15 days (length: + 0.639 mm, volume: + 9.0 mm³) and 30 days (length: + 1.030 mm, volume: +14.2 mm³) of daily use. This result is about 60% higher than the increase of parameters with the placebo. These variations were observed in 87% and 73% of the subjects, respectively. The differences between the areas treated with the active and with the placebo were statistically highly significant.

In addition to these eutrophic effects on the eyelashes, the mascara according to the invention provides a "tonic" effect, observed through the decrease in the number of lashes lost during makeup removal.

## Claims

1. Use of an effective amount of the tripeptide of formula R₁-Gly-His-Lys-R₂ wherein:
- R₁ is an hydrogen atom or an acyl group or a sulfonyl group selected from a biotinoyle or a group comprising an alkyl, aryl, aralkyl, sugar or alkoxy chain of 1 to 24 carbon atoms, which can be linear, branched or cyclic, saturated or unsaturated, hydroxylated or non-hydroxylated, sulfurated or non sulfurated, and
- R₂ is a hydroxyl function or a -O-R₃ group or a -NR₄R₅ group, R₃, R₄ and R₅ being independently of each other hydrogen, an alkyl, aryl, aralkyl, acyl, sulfonyl, sugar or alkoxy chain of 1 to 24 carbons, linear, branched or cyclic, substituted or unsubstituted, saturated or unsaturated, hydroxylated or non-hydroxylated, sulfurated or non sulfurated,
for the topical cosmetic non-therapeutical treatment of eyelashes.

2. Use according to claim 1, wherein the treatment is the improvement of appearance (length and thickness) of eyelashes and their anchorage.

3. Use according to claim 1 or 2, wherein R₁ is biotinoyl and R₂ is hydroxyl (Biot-GHK).

4. Use according to claim 1 or 2, wherein R₁ is palmitoyl (R₁ is an alkyl chain of 16 carbon atomes) and R₂ is hydroxyl (Pal-GHK).

5. Use according to anyone of claims 1 to 4, wherein the tripeptide is combined with an additional active ingredient chosen among :
- Vitamin B complexes, or derivatives, conjugates, homologues or analogs selected from: biotin, pantothenic acid (Vitamin B5), panthenol, pantetheine, pantethine, C1-C30 alkyl esters of pantothenic acid, C1-C30 alkyl esters of the carboxylic acid of panthenol, C1-C30 alkyl ethers of panthenol;
- hyaluronic acid or hyaluronate;
- glucosamine or glycosaminoglycans;
- synthetic analogs of prostaglandins of F-type, preferably the 17-phenyl trinor PGF2α ethyl amide;
- inhibitors of 5-alpha reductase, which help to regulate the activity of sebaceous glands and which reduce the level of sebum in the skin, preferably spironolactone, Zinc PCA or oleanolic acid.

6. Use according to claim 5, wherein the tripeptide is combined with panthenol.

7. Use according to anyone of the preceding claims, wherein the amount of tripeptide is comprised between 0.000001% and 15% by weight, more preferably between 0.0001% and 5% by weight based on the total weight of the composition.

8. Use according to claim 6 or 7, wherein it comprises an amount of panthenol comprised between 0.001 % and 5% by weight based on the total weight of the composition.

## Patentansprüche

1. Verwendung einer wirksamen Menge des Tripeptids der Formel R₁-Gly-His-Lys-R₂, wobei:
- R₁ ein Wasserstoffatom oder eine Acylgruppe oder eine Sulfonylgruppe ist, ausgewählt aus einem Biotinoyl oder einer Gruppe umfassend ein Alkyl, Aryl, Aralkyl, Zucker oder eine Alkoxykette aus 1 bis 24 Kohlenstoffatomen, die unverzweigt, verzweigt oder cyclisch, gesättigt oder ungesättigt, hydroxyliert oder nicht hydroxiliert, schwefelhaltig oder nicht schwefelhaltig sein kann, und
- R₂eine Hydroxylfunktion oder eine -O-R₃-Gruppe oder eine -NR₄R₅-Gruppe ist, wobei R₃, R₄ und R₅ unabhängig voneinander Wasserstoff, ein Alkyl, Aryl, Aralkyl, Acyl, Sulfonyl, Zucker oder Alkoxykette aus 1 bis 24 Kohlenstoffen, unverzweigt, verzweigt oder cyclisch, substituiert oder unsubstituiert, gesättigt oder ungesättigt, hydroxyliert oder nicht hydroxiliert, schwefelhaltig oder nicht schwefelhaltig sind, für die topische kosmetische, nicht therapeutische Behandlung von Wimpern.

2. Verwendung nach Anspruch 1, wobei die Behandlung aus der Verbesserung des Aussehens (Länge und Dicke) der Wimpern und deren Fixierung besteht.

3. Verwendung nach Anspruch 1 oder 2, wobei R₁ Biotinoyl ist und R₂ Hydroxyl (Biot-GHK) ist.

4. Verwendung nach Anspruch 1 oder 2, wobei R₁ Palmitoyl ist (R₁ ist eine Alkylkette aus 16 Kohlenstoffatomen) und R₂ Hydroxyl (Pal-GHK) ist.

5. Verwendung nach einem der Ansprüche 1 bis 4, wobei das Tripeptid mit einem zusätzlichen Wirkstoff kombiniert ist, ausgewählt aus:
- Vitamin-B-Komplexen oder Derivaten, Konjugaten, Homologen oder Analoga, ausgewählt aus: Biotin, Pantothensäure (Vitamin B5), Panthenol, Pantethein, Pantethin, C1-C30-Alkylester von Pantothensäure, C1-C30-Alkylester der Carbonsäure von Panthenol, C1-C30-Alkylether von Panthenol;
- Hyaluronsäure oder Hyaluronat;
- Glucosamin oder Glycosaminoglycanen;
- synthetischen Analoga von Prostaglandinen vom F-Typ, vorzugsweise das 17-Phenyltrinor-PGF2α-ethylamid;
- Inhibitoren der 5-alpha-Reduktase, die dabei helfen, die Aktivität der Talgdrüsen zu regulieren und den Talgspiegel in der Haut reduzieren, vorzugsweise Spironolacton, Zink-PCA oder Oleanolsäure.

6. Verwendung nach Anspruch 5, wobei das Tripeptid mit Panthenol kombiniert ist.

7. Verwendung nach einem der vorhergehenden Ansprüche, wobei die Menge an Tripeptid zwischen 0,000001 Gew.-% und 15 Gew.-%, bevorzugter zwischen 0,0001 Gew.-% und 5 Gew.-% auf Basis des Gesamtgewichts der Zusammensetzung liegt.

8. Verwendung nach Anspruch 6 oder 7, wobei sie eine Menge an Panthenol umfasst, die zwischen 0,001 Gew.-% und 5 Gew.-% auf Basis des Gesamtgewichts der Zusammensetzung liegt.

## Revendications

1. Utilisation d'une quantité efficace du tripeptide de formule R₁-Gly-His-Lys-R₂ dans laquelle :
- R₁ est un atome d'hydrogène ou un groupement acyle ou sulfonyle choisi parmi un groupement biotinoyle ou un groupement comportant une chaîne alkyle, aryle, aralkyle, sucre ou alkoxy de 1 à 24 atomes de carbone, pouvant être linéaire, ramifiée ou cyclique, saturée ou insaturée, hydroxylée ou non-hydroxylée, soufrée ou non soufrée, et
- R₂ est une fonction hydroxyle ou un groupement -O-R₃ ou un groupement -NR₄R₅, R₃, R₄ et R₅ étant indépendamment les uns des autres un hydrogène, une chaine alkyle, aryle, aralkyle, acyle, sulfonyle, sucre ou alkoxy de 1 à 24 carbones, linéaire, ramifiée ou cyclique, substituée ou non-substituée, saturée ou insaturée, hydroxylée ou non hydroxylée, soufrée ou non soufrée,
pour le traitement topique cosmétique non thérapeutique des cils.

2. Utilisation selon la revendication 1, **caractérisée en ce que** le traitement consiste en l'amélioration de l'apparence (longueur et épaisseur) des cils et leur ancrage.

3. Utilisation selon la revendication 1 ou 2, **caractérisée en ce que** R₁ est un biotinoyle et R₂ est un hydroxyle (Biot-GHK).

4. Utilisation selon la revendication 1 ou 2, **caractérisée en ce que** R₁ est un palmitoyle (R₁ est une chaîne alkyle à 16 atomes de carbone) et R₂ est un hydroxyle (Pal-GHK).

5. Utilisation selon l'une des revendications 1 à 4, **caractérisé en ce que** le tripeptide est combiné à un ingrédient actif additionnel choisi parmi :
- les complexes de Vitamine B, ou dérivés, conjugués, homologues ou analogues choisis parmi : la biotine, l'acide pantothénique (vitamine B5), le panthénol, la pantéthéine, la pantéthine, les esters alkylés en C1-C30 de l'acide pantothénique, les esters alkylés en C1-C30 de l'acide carboxylique de panthénol, les éthers alkylés en C1-C30 de panthénol ;
- l'acide hyaluronique ou hyaluranate ;
- la glucosamine ou glycosaminoglycane ;
- des analogues de synthèse de prostaglandines de type F, préférentiellement le 17-phényl trinor PGF2α éthyl amide ;
- des inhibiteurs de 5-alpha-réductase, aidant à réguler l'activité des glandes sébacées et réduisant le niveau de sébum de la peau, préférentiellement la spironolactone, le Zinc PCA ou l'acide oléanolique.

6. Utilisation selon la revendication 5, **caractérisée en ce que** le tripeptide est combiné avec le panthénol.

7. Utilisation selon l'une quelconque des revendications précédentes, **caractérisée en ce que** la quantité de tripeptide est comprise entre 0,000001% et 15% en poids, de préférence encore entre 0,0001% et 5% en poids par rapport au poids total de la composition.

8. Utilisation selon l'une des revendications 6 ou 7, **caractérisée en ce qu'**elle contient une quantité de Panthénol comprise entre 0,001% à 5% en poids par rapport au poid total de la composition.


### SEQUENCE LISTING

<110> SEDERMA
   FOURNIAL, ARNAUD
   BERNARD, GERARD
<120> NEW COSMETIC AND THERAPEUTICAL USES OF GHK TRIPEPTIDE
<130> HAIRGRO PCT
<150> FR1153470
   <151> 2011-04-21
<160> 10
<170> PatentIn version 3.5
<210> 1
   <211> 4
   <212> PRT
   <213> artificial sequence
<220>
   <223> synthetic peptide
<220>
   <221> MOD_RES
   <222> (1)..(1)
   <223> amidation by a Palmitoyl chain on the N terminal end
<400> 1
   Gly Gln Pro Arg
   1
<210> 2
   <211> 5
   <212> PRT
   <213> artificial sequence
<220>
   <223> synthetic peptide
<400> 2
   Lys Thr Thr Lys Ser
   1               5
<210> 3
   <211> 5
   <212> PRT
   <213> artificial sequence
<220>
   <223> synthetic peptide
<220>
   <221> MOD_RES
   <222> (1)..(1)
   <223> amidation by a Palmitoyl chain on the N terminal end
<400> 3
   Lys Thr Thr Lys Ser
   1               5
<210> 4
   <211> 4
   <212> PRT
   <213> artificial sequence
<220>
   <223> synthetic peptide
<400> 4
   Arg Ser Arg Lys
   1
<210> 5
   <211> 4
   <212> PRT
   <213> artificial sequence
<220>
   <223> synthetic peptide
<400> 5
   Gly Gln Pro Arg
   1
<210> 6
   <211> 4
   <212> PRT
   <213> artificial sequence
<220>
   <223> synthetic peptide
<400> 6
   Lys Thr Phe Lys
   1
<210> 7
   <211> 6
   <212> PRT
   <213> artificial sequence
<220>
   <223> Synthetic peptide
<400> 7
   Gly Lys Thr Thr Lys Ser
   1 5
<210> 8
   <211> 6
   <212> PRT
   <213> artificial sequence
<220>
   <223> synthetic peptide
<400> 8
   Val Gly Val Ala Pro Gly
   1 5
<210> 9
   <211> 5
   <212> PRT
   <213> artificial sequence
<220>
   <223> synthetic peptide
<220>
   <221> MOD_RES
   <222> (1)..(1)
   <223> amidation by a Palmitoyl chain on the N terminal end
<220>
   <221> MISC_FEATURE
   <222> (5)..(5)
<220>
   <221> MISC_FEATURE
   <222> (5)..(5)
   <223> Xaa being either a Methionine M or a Leucine L.
<400> 9
   Tyr Gly Gly Phe Xaa
   1 5
<210> 10
   <211> 6
   <212> PRT
   <213> artificial sequence
<220>
   <223> synthetic peptide
<220>
   <221> MOD_RES
   <222> (1)..(1)
   <223> amidation by a Palmitoyl chain on the N terminal end
<400> 10
   Val Gly Val Ala Pro Gly
   1 5